# EUROPEAN PATENT SPECIFICATION

(11) **EP 4 415 819 B1**
(45) Date of publication and mention of the grant of the patent: **29.07.2026**
(21) Application number: 22809554.3
(22) Date of filing: 13.10.2022
(51) Int. Cl.: A61P 3/10, C07C 53/10, C07C 237/04

(54) **MONOHYDRATE SALT OF DENATONIUM ACETATE**
MONOHYDRATSALZ VON DENATONIUMACETAT
SEL MONOHYDRATE D'ACÉTATE DE DÉNATONIUM

(30) Priority: 14.10.2021 US 202163255947 P
(43) Date of publication of application: 21.08.2024
(73) Proprietor: Aardvark Therapeutics Inc., San Diego, CA 92121 (US)
(72) Inventor: OSTER, Jeffrey, San Diego, California 92121 (US); ZEILER, Andrew, San Diego, California 92121 (US)
(74) Representative: Maiwald GmbH
(86) International application number: PCT/US2022/046585
(87) International publication number: WO 2023/064480

(56) References cited:
- WO-A1-2021/062061
- WO-A1-2021/133908

## Description

### Cross-Reference to Related Applications

This application claims the benefit of priority of US Provisional Application No. 63/255,947, filed October 14, 2021.

### Technical Field

The present disclosure provides a novel crystalline monohydrate salt form of denatonium acetate. More particularly, the present novel salt form and crystalline hydrate form is useful for the treatment and prevention of diseases and conditions. In particular, such diseases and conditions include metabolic syndrome, obesity, NASH, glycemic control/diabetes, Prader Willi Syndrome and IBD (intestinal bowel disease).

### Background

Denatonium acetate is a salt mentioned in US2015/0252305 ("The bittering agent is preferably a denatonium salt or a derivative thereof. In one aspect, the bittering agent is a denatonium salt selected from the group consisting of denatonium chloride, denatonium citrate, denatonium saccharide, denatonium carbonate, denatonium acetate, denatonium benzoate, and mixtures thereof. In one aspect, the liquid composition comprises a first denatonium salt and the film comprises a second denatonium salt that is different than the first denatonium salt."). However, it appears denatonium acetate was never synthesized but listed only as a theoretical salt of many with denatonium as the cation. Later, a group of patent applications, assigned to Aardvark Therapeutics, Inc., described denatonium acetate as a pharmaceutical composition and included a synthesis process for synthesizing denatonium acetate from lidocaine. See, e.g., WO2021/062061. That synthesis process produces denatonium acetate, anhydrous (DAA). However, it was subsequently determined that DAA was insufficiently stable and was not suitable for use as an API (active pharmaceutical ingredient) as there are FDA regulatory requirements for stability of active pharmaceutical ingredients that DAA did not meet. Therefore, there is a need in the art for a more stable salt form of denatonium acetate (DA). Denatonium acetate is known to be useful for treating various conditions such as metabolic syndrome, obesity, and hyperglycemia. See, e.g., WO2021/133908.

### Summary

The present disclosure is based on the discovery that DAA is unstable and degrades under at least some conditions. DAA was hydrated and recrystallized into a stable salt, i.e., denatonium acetate monohydrate (DAM). The present disclosure provides a novel denatonium acetate monohydrate salt and crystalline forms thereof. The denatonium acetate monohydrate salt and crystalline forms can provide advantages in the preparation of denatonium acetate, such as greater stability, handling and dosing. In particular, DAM can exhibit improved physical and chemical stability to stress, high temperatures and humidity, e.g., relative to DAA.

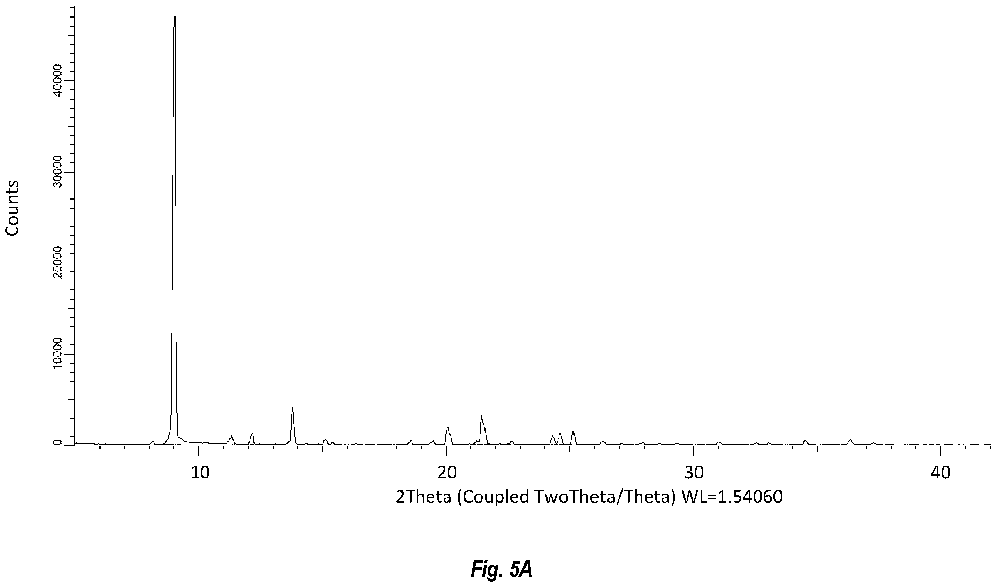

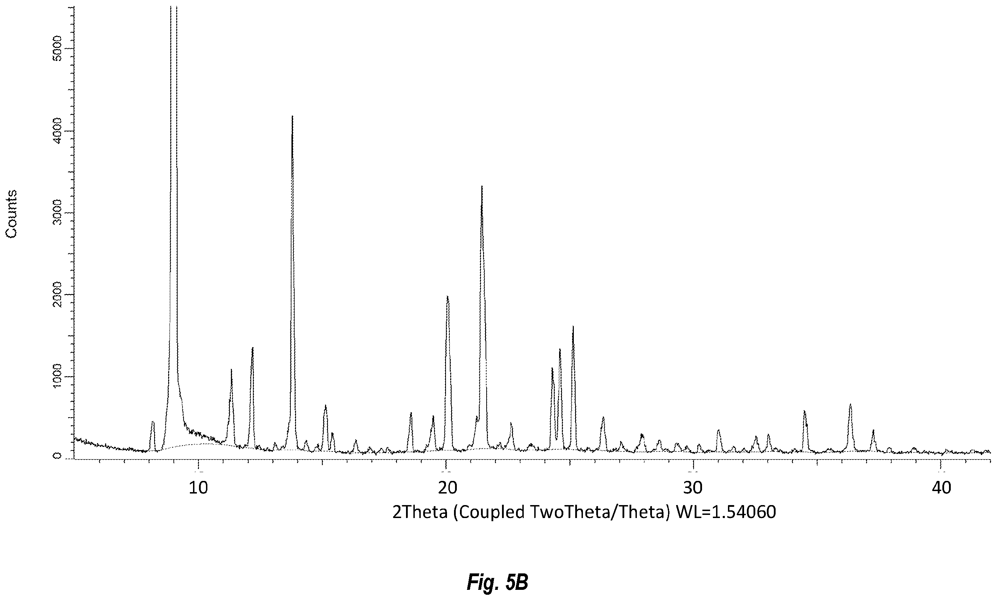

The present disclosure provides a crystalline denatonium acetate monohydrate salt of structural formula 1: 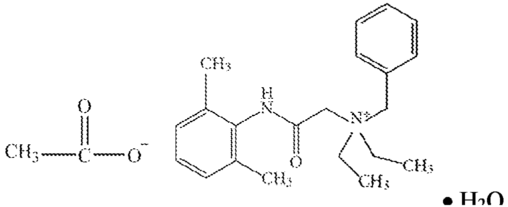 The salt may be characterized by an X-ray powder diffraction (XRPD) spectrum substantially as shown in Fig. 5A or Fig. 5B.

The present disclosure further provides a pharmaceutical composition comprising a therapeutically effective amount of the denatonium acetate monohydrate crystalline salt in association with one or more pharmaceutically acceptable carriers. The one or more pharmaceutically acceptable carriers may comprise a biocompatible polymer. The biocompatible polymer may be cellulose. The one or more pharmaceutically acceptable carriers may comprise a saccharide. The saccharide may be a sugar alcohol. The sugar alcohol may be mannitol. The one or more pharmaceutically acceptable carriers may comprise talc. The one or more pharmaceutically acceptable carriers comprise an organic acid. The organic acid may be acetic acid. The pharmaceutical composition may be formulated for oral administration. The pharmaceutical composition may comprise solid granules.

And the present disclosure provides a process for preparing denatonium acetate monohydrate crystalline salt comprising the steps of contacting an equivalent of DAA (denatonium acetate, anhydrous) with a lower alkyl isobutyl ketone, such as methyl isobutyl ketone, and water such that the water concentration is above 10 weight percent, recovering the resultant solid phase, and removing the solid therefrom. The present disclosure provides a process for preparing a denatonium acetate monohydrate salt described herein comprising contacting anhydrous denatonium acetate with a lower alkyl isobutyl ketone and water, resulting in formation of a solid phase comprising denatonium acetate monohydrate. The lower alkyl isobutyl ketone may be methyl isobutyl ketone or ethyl isobutyl ketone. Contacting the anhydrous denatonium acetate with a lower alkyl isobutyl ketone and water forms a composition in which the water concentration is above 10 weight percent. The denatonium acetate monohydrate is a crystalline monohydrate. The crystalline monohydrate may be characterized by an X-ray powder diffraction (XRPD) spectrum substantially as shown in Fig. 5A or Fig. 5B.

### Brief Description of the Figure

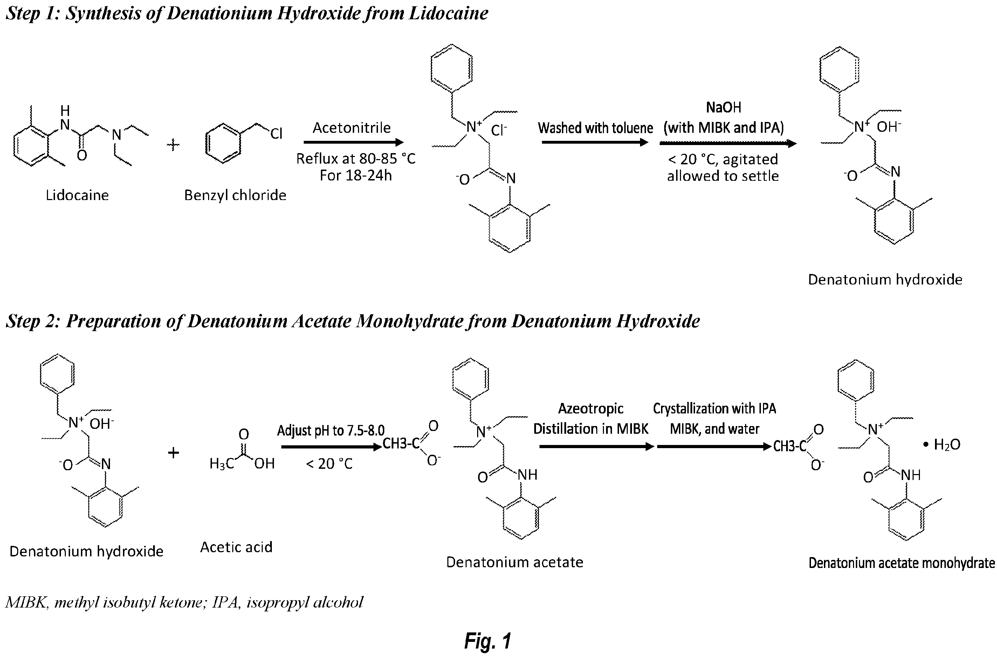

Figure 1 shows a synthesis process for making DAM from DAA.
Figure 2 shows a form diagram showing the relationship between the polymorph patterns found for DAM.
Figure 3 shows an overlay diffractogram of DAM of Pattern 1 (lower trace) and Pattern 2 (upper trace).
Figure 4 shows an overlay diffractogram of DAM of Pattern 1 (lower trace) and Pattern 5 in red (upper trace).
Figure 5A shows a diffractogram of DAM of Pattern 1.
Figure 5B shows an enlarged version of the region of the diffractogram of DAM of Pattern 1 from 0 to approximately 5500 counts.

### Detailed Description

A process for synthesizing DAM begins with Lidocaine base to make Denatonium Hydroxide and then DAA.

### Step 1: Synthesis of Denatonium Hydroxide from Lidocaine

To a reflux apparatus add 25 g of lidocaine, 60 ml of water, and 17.5 g of benzyl chloride with stirring and heating in 70-90 °C. The solution needs to be heated and stirred in the before given value for 24h, the solution needs to be cooled down to 30 °C. The unreacted reagents are removed with 3×10 mL of toluene. With stirring dissolve 65 g of sodium hydroxide into 65 mL of cold water and add it to the aqueous solution with stirring over the course of 3 h. Filter the mixture, wash with some water and dry in open air. Recrystallize in hot chloroform or hot ethanol.

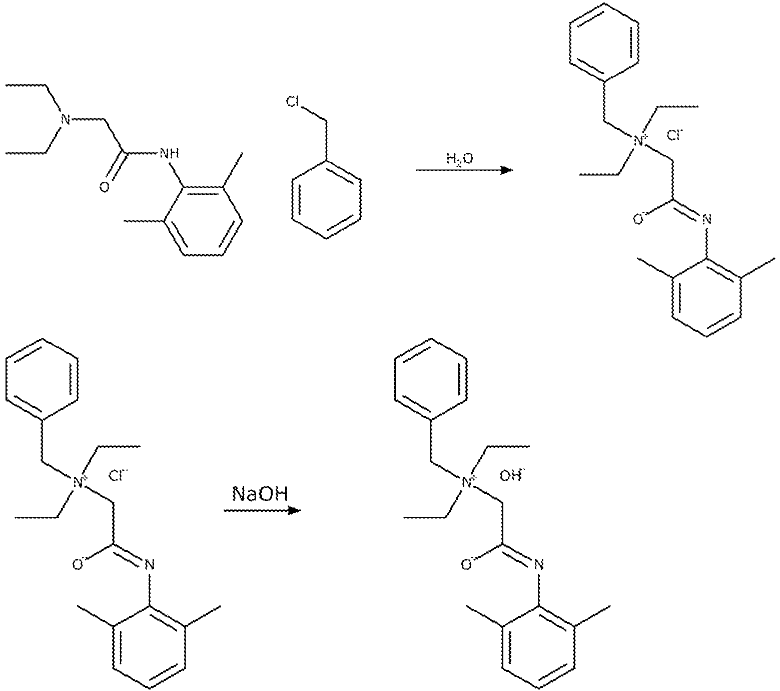

### Step 2: Preparation of Denatonium Acetate, anhydrous (DAA) from Denatonium Hydroxide.

To a reflux apparatus 10 g of denatonium hydroxide (MW: 342.475 g/mol, 0.029 mol), 20 mL of acetone, and 2 g of acetic acid glacial (0.033 mol) dissolved in 15 mL of acetone is added, the mixture is stirred and heated to 35 °C for 3 h. Then evaporated to dryness and recrystallized in hot acetone.

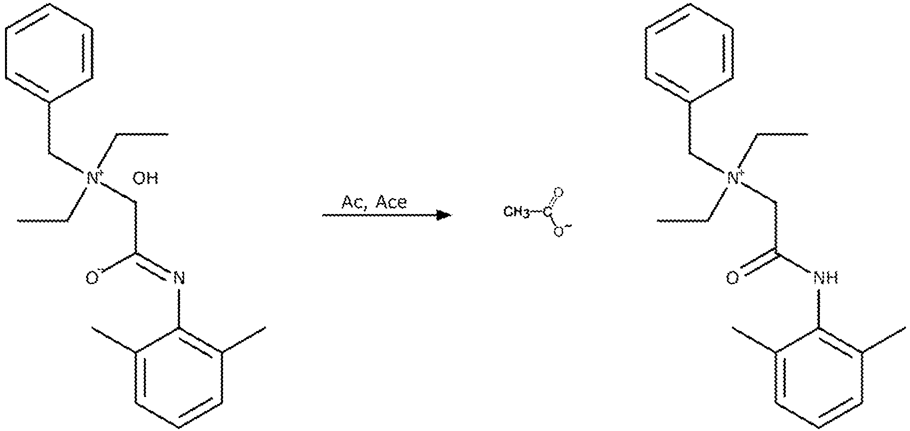

The DAA degraded and it was found that DAA degraded to either (A) lidocaine and benzyl acetate or it degraded to (B) 2-(diethylamino)-3-phenyl-N-(2,6-dimethylphenylpropionamide. Therefore, DAA required a low temperature to be used as an intermediate. For Step 3, DAA was maintained in an organic layer and was distilled under vacuum until the temperature reached 65-67 °C at <150 torr (20 kPa). Methyl isobutyl ketone was added and refluxed under vacuum to remove water via azeotropic distillation to form DAM. DAM was crystalized by adding isopropyl alcohol. Residual salts were removed. The mixture was distilled under vacuum. Next, methyl isobutyl ketone was added and then water. In some embodiments, a lower alkyl isobutyl ketone is used in place of methyl isobutyl ketone. In some embodiments, the lower alkyl is a C₁₋₃ alkyl. In some embodiments, the lower alkyl is methyl or ethyl. The temperature was lowered to <10 °C. The remaining solid was isolated and washed with methyl isobutyl ketone to produce the final DAM (denatonium acetate monohydrate).

The present disclosure provides crystalline denatonium acetate monohydrate. In some embodiments, the crystalline denatonium acetate monohydrate is characterized by an X-ray powder diffraction (XRPD) spectrum substantially as shown in Fig. 5A or Fig. 5B.

In some embodiments, a pharmaceutical composition is provided comprising denatonium acetate monohydrate and a pharmaceutically acceptable carrier. The pharmaceutically acceptable carrier may comprise, e.g., a biocompatible polymer such as cellulose and/or a saccharide, e.g., a sugar alcohol such as mannitol. In some embodiments, the pharmaceutically acceptable carrier comprises talc. In some embodiments, the pharmaceutical acceptable carrier comprises talc, a cellulose and a saccharide. In some embodiments, the pharmaceutical composition is an oral formulation. In some embodiments, the pharmaceutical composition (e.g., an oral formulation) is a sustained release cellulosic and mannitol excipient formulation. In some embodiments, the pharmaceutically acceptable carrier comprises an organic acid, such as acetic acid, which may be present in combination with any of the foregoing components or combinations of components. The pharmaceutical composition may be formulated for oral administration. The pharmaceutical composition may comprise solid granules.

### Example 1

This example describes the characterization of a batch of Denatonium acetate monohydrate (DAM) and polymorph screening studies carried out with the material. DAM was highly crystalline with an XRPD Pattern designated as Pattern 1 (Fig. 5A; enlarged version in Fig. 5B).

Gravimetric Vapor Sorption (GVS) experiments and storage under stress conditions performed on DAM show that the material was very hygroscopic at relative humidity (RH) levels above 80%. It was observed to deliquesce at 96% RH after 5 days. It also lost mass when taken to relative humidity levels of less than 10%. A sample of DAM kept at 0% RH converted to Pattern 5. This pattern is metastable and converted to Pattern 1 under ambient laboratory conditions within a few hours.

DAM was thermally unstable when heated to 150 °C (just beyond the melt) in the DSC showing only ca. 54.9% of input material remaining by UPLC analysis.

Attempts to prepare amorphous material by lyophilization from a range of solvents, by fast evaporation or anti-solvent precipitation did not yield amorphous material. Pattern 1, as supplied, was used as input material for the polymorph screening studies using 48 solvents, as well as the solvent/anti-solvent screening studies. During all the investigations into DAM there were 4 additional XRPD Patterns found.

Pattern 2 was obtained from the attempted preparation of amorphous material by lyophilization of a solution of DAM in t-butanol. After storage for 7 weeks in a closed vial it was observed to have converted to Pattern 1.

Pattern 3 (from evaporation of a solution of DAM in ethyl formate) was found to be a mixture of acetate and formate salts of denatonium based upon the ¹H NMR and is therefore not a pattern representative of denatonium acetate alone.

Pattern 4 was obtained from the polymorph screen by evaporation of a solution of DAM in 2-methoxyethanol. A sample reanalyzed by XRPD after storage in a closed vial for 6 weeks shows conversion to Pattern 1.

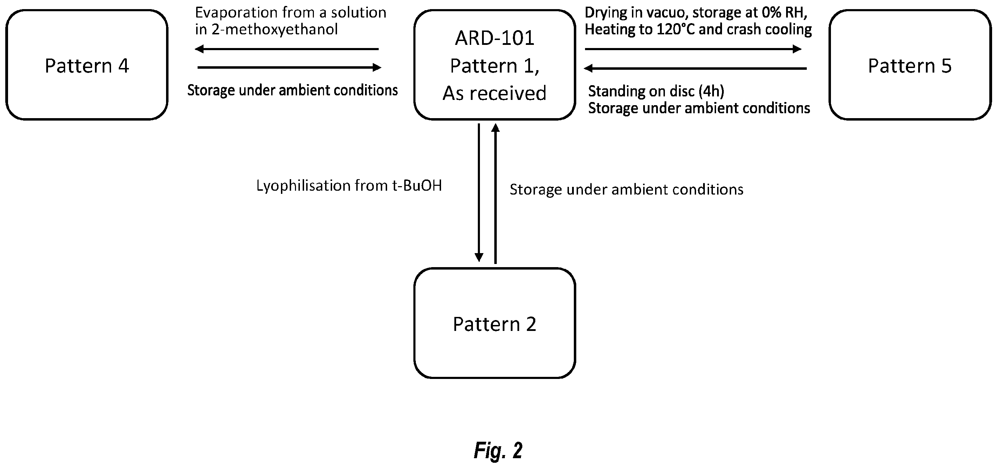

Pattern 5 was obtained by storage of DAM at 0% RH, drying *in vacuo* at room temperature or heating to 120 °C in the DSC and crash cooling. It was observed to readily convert to Pattern 1 on standing on the XRPD disc under ambient lab conditions after 4 hours. A form diagram showing the relationship between the patterns found can be seen in Figure 2.

A summary of the initial characterization of DAM is provided in Table 1.

**Table 1**

| | |
|---|---|
| CRL Batch reference | ED21356-001-001-00 |
| Supplier batch reference | DAM (ARD19G01-P) |
| Compound name | Denatonium acetate monohydrate |
| Appearance | White solid |
| ¹H NMR | Consistent with structure. |
| UPLC purity | 99.5%, shows [M]⁺ 325.4 consistent with C₂₁H₂₉N₂O⁺ |
| XRPD | Assigned as Pattern 1 (Highly crystalline) |
| DSC | DSC of ED21356-001-001-00 at 10 °C/min shows a broad shallow endothermic event between 44-124 °C, a broad endothermic event of onset 135 °C consistent with a possible melt and further endothermic events between 149-206 °C |
| TGA | TGA of ED21356-001-001-00 at 10 °C/min shows mass losses of 4.3% over 40-153 °C, 12.3% over 154-192 °C, 7.8% over 193-220 °C followed by further mass losses |
| PSD | D₁₀ 1.62 µm, D₅₀ 23.09 µm, D₉₀ 65.07 µm |

Particle size distribution was measured and showed a bimodal distribution with a D₉₀ of *ca.* 65 µm. ES+ mass spectrum of DAM shows a M+ ion of 325.4 consistent with the quaternary component. The sample was highly crystalline. Thermal analysis of DAM by Differential Scanning Calorimetry (DSC) at 10 °C/min showed a broad shallow endothermic event between 44-124 °C, a broad endothermic event of onset 135 °C consistent with a possible melt and further endothermic events between 149-206 °C. Thermogravimetric analysis (TGA) at 10 °C/min showed mass losses of 4.3% over 40-153 °C, 12.3% over 154-192 °C, 7.8% over 193-220 °C followed by further mass losses.

Several Gravimetric Vapor Sorption (GVS) experiments were carried out to investigate the behavior of DAM over a range of different relative humidity levels. A GVS experiment (GVS 1) over the relative humidity range (40-90-0-90-0-40%) showed DAM to be very hygroscopic with a 23% mass increase over the 0-90% relative humidity range (second sorption cycle). Most of the mass increase was observed to take place above 80% RH. It is worth noting that the experiment did not reach equilibrium at the highest humidity stages (even with a method allowing for a maximum of 24 hours at each stage if equilibrium is not reached) and so the mass increase of 23% was likely to be an under estimate of the hygroscopicity.

A GVS experiment (GVS 2) with DAM (DAM, lot:ED21356-001-001-00) to investigate the behavior of the material at levels of relative humidity up to 80% was performed. This experiment used a similar method to GVS 1 without the steps involving 90% RH where the large mass changes were observed and equilibrium was not reached. GVS 2 over the relative humidity range (40-80-0-80-0-40%) starting with a sorption cycle showed an increase in mass of 2.1% (over 0-80% relative humidity in the second sorption cycle) with most of this mass change (+1.4%) taking place between 0-10% RH. The material remained as a free-flowing powder after the experiment and XRPD analysis showed a diffractogram consistent with Pattern 1.

A further GVS experiment (GVS 3) with DAM over the relative humidity range (40-0-80-0-80-0-40%) was performed to investigate whether there was a significant difference in behavior if the sample had not been exposed to high (80%) RH before the start of the 0-80% RH sorption cycle. This experiment (GVS 3) starting with a desorption cycle showed an increase in mass of 2% (over 0-80% relative humidity in both the first and second sorption cycles). This was the same for both sorption cycles in the experiment and comparable with the result of 2.1% obtained in GVS 2. Most of the mass change (1.3 - 1.4% increase) occurred between 0-10% RH. The material remained as a free-flowing powder after the experiment and XRPD analysis showed a diffractogram consistent with Pattern 1.

The GVS experiments and storage under stress conditions carried out with DAM show that the material is very hygroscopic at relative humidity levels above 80%. It showed a mass increase of 1.4% between 0-10% RH and 2.1% between 0-80% RH (in GVS 2) and 23% (in GVS 1) between 0-90% RH. It was observed to deliquesce after storage at 96% RH after 5 days. It also loses mass when taken to relative humidity levels of less than 10%.

After the observation that DAM lost mass when taken from 10% RH to 0% RH in the GVS experiments described above, a portion of DAM was placed in an open vial and kept at 0% RH in the GVS instrument for 2 days. It was then removed from the instrument and a portion immediately analyzed by XRPD. It showed a diffractogram consistent with Pattern 5. Reanalysis of the sample after standing on the disc for 4 hours under ambient laboratory conditions showed conversion to Pattern 1.

The GVS and storage under stress conditions experiments carried out with DAM show that the material is very hygroscopic at relative humidity levels above 80%. It also loses mass when taken to relative humidity levels of less than 10%. A sample of DAM kept at 0% RH converted to Pattern 5, however this Pattern is metastable and converts to Pattern 1 under ambient laboratory conditions within a few hours.

A series of additional DSC experiments were carried out to further investigate the thermal behavior of DAM. A sample of DAM (5 mg) was heated in the DSC at 10 °C/min to 150 °C and then cooled to 30 °C. After the experiment the pan was removed from the instrument, the pan opened and the material (DAM, lot:ED21356-003-001-00) was investigated to check for signs of degradation. UPLC shows significant signs of degradation with only *ca.* 54.9% of input material remaining. The ¹H NMR also shows evidence of substantial degradation.

A sample of DAM (4.55 mg) was heated in the DSC at 10 °C/min to 120 °C, held at 120 °C for 2 minutes and then cooled to 30 °C. After the experiment the pan was removed from the instrument, the pan opened and the material was investigated to check for signs of degradation. UPLC and ¹H NMR showed no significant signs of degradation.

The DSC experiments carried out with DAM show that the material is thermally unstable when heated to 150 °C showing significant decomposition by NMR and UPLC. No significant chemical degradation was observed when the sample was heated briefly to 120 °C, however a change in XRPD diffractogram from Pattern 1 to Pattern 5 was observed. The material reverted to Pattern 1 after standing at room temperature overnight. Pattern 5 is metastable and converts to Pattern 1 under ambient laboratory conditions after standing overnight on the XRPD disc. A summary of the initial and further characterization of DAM is provided in Table 2.

**Table 2**

| | |
|---|---|
| CRL Batch reference | ED21356-001-001-00 |
| Supplier batch reference | DAM (ARD19G01-P) |
| Compound name | Denatonium acetate monohydrate |
| Appearance | White solid |
| ¹H NMR | Consistent with structure. |
| UPLC purity | 99.5%, shows [M]⁺ 325.4 consistent with C₂₁H₂₉N₂O⁺ |
| XRPD | Assigned as Pattern 1 (Highly crystalline) |
| DSC | DSC of ED21356-001-001-00 at 10 °C/min shows a broad shallow endothermic event between 44-124 °C, a broad endothermic event of onset 135 °C consistent with a possible melt and further endothermic events between 149-206 °C |
| TGA | TGA of ED21356-001-001-00 at 10 °C/min shows mass losses of 4.3% over 40-153 °C, 12.3% over 154-192 °C, 7.8% over 193-220 °C followed by further mass losses |
| PSD | D₁₀ 1.62 µm, D₅₀ 23.09 µm, D₉₀ 65.07 µm |
| GVS 1 (40-90-0-90-0-40) | ED21356-001-001-00 is very hygroscopic especially at levels of RH above 80%. Shows a 23% mass increase over second sorption cycle. Sample had formed a solid mass with powdery material in centre. |
| GVS 2 (40-80-0-80-0-40) | Shows a 2.1% mass increase over second sorption cycle. Sample remained free flowing solid |
| GVS 3 (40-0-80-0-80-0-40) | Shows a 2% mass increase over first and second sorption cycles |
| | Sample remained free flowing solid |
| XRPD post GVS | Shows no change in form by XRPD post GVS 1,2,3 |
| Storage at 25 °C, 96% RH 5 days | Sample had deliquesced |
| Storage at 25 °C, 96% RH (ED21356-001-001-03) 7 days | Sample had deliquesced. (Amorphous by XRPD) UPLC purity 98.9% |
| Storage at 40 °C, 75% RH (ED21356-001-001-02) 7 days | Remained a solid after 5 and 7 days. |
| | Shows no change in form by XRPD after 7 days UPLC purity 99.4% |
| Storage at 0% RH | Shows a change in form to Pattern 5, this reverted to Pattern 1 on standing for 4 hours on disc |
| Heat to 150 °C in DSC | Shows significant decomposition. UPLC purity 54.9% |
| Heat to 120 °C in DSC | Shows no significant chemical decomposition |
| Heat to 120 °C in DSC, crash cool and then heat to 300 °C | First heating cycle shows a broad endothermic event between 56 °C and 106 °C. This is not present in the second heating cycle. |
| Heat to 120 °C in DSC, crash cool and XRPD | Shows a change in form to Pattern 5, this reverted to Pattern 1 after standing overnight |

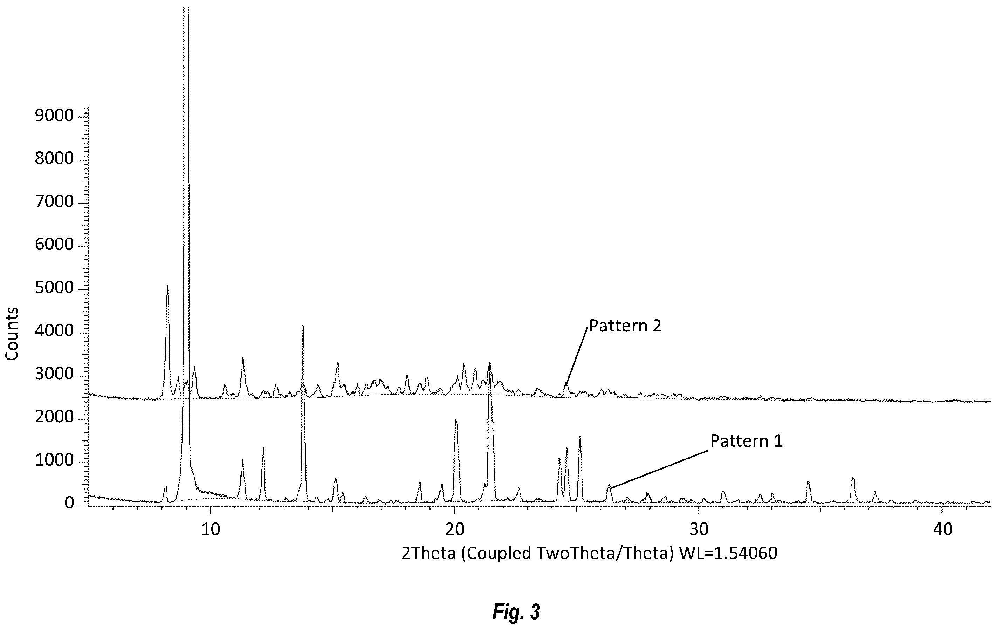

A portion of DAM (8 mg) was completely dissolved in t-butanol (0.5 mL) and then placed in dry ice to freeze. The frozen sample was lyophilized on the freeze dryer until all the solvent was removed. The resulting solid material (DAM, lot:ED21356-005-001-01) was analyzed by XRPD. It was not amorphous and showed a different pattern to the input. This was designated as Pattern 2. Figure 3 shows an overlay diffractogram of DAM of Pattern 1 in black and Pattern 2 in red. The sample of Pattern 2 was reanalyzed by XRPD after storage in a closed vial for 7 weeks and showed a diffractogram consistent with Pattern 1.

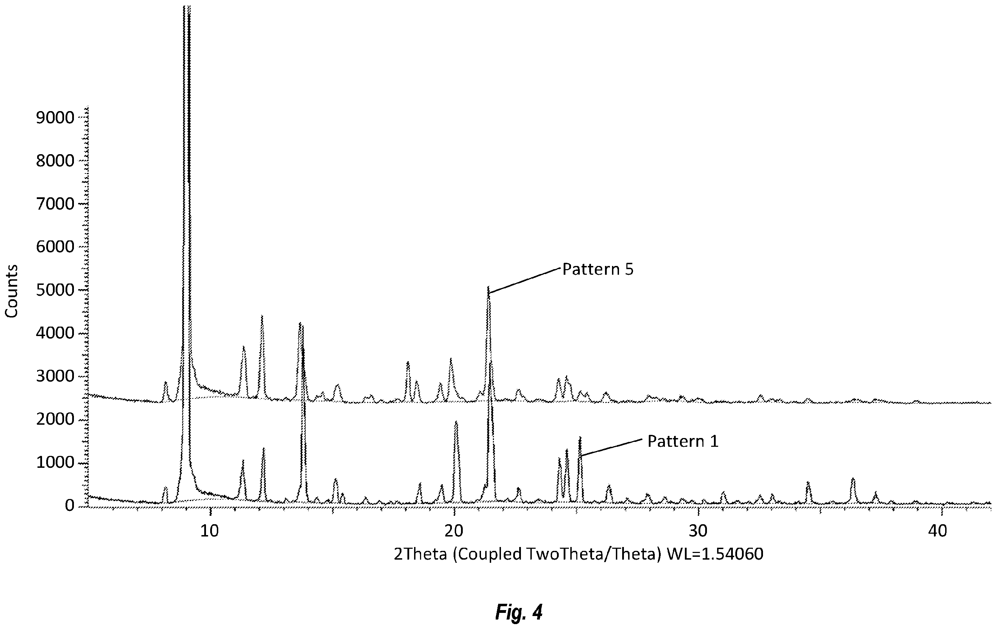

A portion of DAM (10.9 mg), was dried at room temperature overnight *in vacuo,* to give a solid ED21356-004-001-00. It was analyzed by XRPD and was found not to be amorphous. It showed diffraction peaks consistent with crystalline material. It showed a pattern quite similar to Pattern 1 with an extra peak at 18° 2θ and some other small differences as shown in the overlay in Figure 4. This was assigned as Pattern 5.

Attempts to prepare amorphous DAM by drying *in vacuo,* fast evaporation from DCM, anti-solvent precipitation or freeze drying from water, 1,4-dioxane/water (1: 1), MeCN/water (1:1) or t-Butanol under the conditions of these studies did not give amorphous material. Crystalline material consistent with Pattern 1 was obtained from fast evaporation from DCM or freeze drying from water, 1,4-dioxane/water (1: 1) or MeCN/water (1:1). The rapid addition of a concentrated solution of DAM in DCM to heptane resulted in precipitation. Analysis of the solid by XRPD showed diffraction peaks of DAM Pattern 1. In the case of freeze drying from t-BuOH a new Pattern, designated Pattern 2, was isolated. This was found to have converted to Pattern 1 after storage in a closed vial after 7 weeks.

Drying of a sample of DAM *in vacuo* at room temperature gave Pattern 5. This was found to have converted to Pattern 1 after storage in a closed vial after 6 weeks.

Attempts were made to crystallize the API (DAM) from a wide range of solvent systems to try to generate new crystalline forms. DAM was used as input material for these screening studies and solvents chosen were mainly ICH class II and III with a range of different properties as shown in Table 3 below.

**Table 3**

| Solvent | Boiling Point °C | ICH Class | Solvent | Boiling Point °C | ICH Class |
|---|---|---|---|---|---|
| Diethyl ether | 35 | III | Propyl acetate | 102 | III |
| Pentane | 36 | III | 2-Pentanone | 105 | no data |
| Ethyl formate | 53 | III | 2-Methyl-1-propanol | 108 | III |
| t-Butylmethyl ether | 55 | III | Toluene | 111 | II |
| Acetone | 56 | III | Isobutyl acetate | 116 | III |
| Methyl acetate | 58 | III | Methylisobutyl ketone | 117 | II |
| Chloroform | 61 | II | 1-Butanol | 118 | III |
| methanol | 65 | II | Acetic acid | 118 | III |
| Tetrahydrofuran | 66 | II | 2-Methoxyethanol | 124 | II |
| Diisopropyl ether | 69 | no data | Butyl acetate | 125 | III |
| Ethyl acetate | 77 | III | Methylbutyl ketone | 127 | II |
| Ethanol | 78 | III | 3-Methyl-1-Butanol | 130 | III |
| Methylethyl ketone | 80 | III | 2-Ethoxyethanol | 135 | II |
| Acetonitrile | 81 | II | 1-Pentanol | 137 | III |
| 2-Propanol | 82 | III | Cumene | 152 | II |
| t-Butanol | 82 | II | Anisole | 154 | III |
| 1,2-Dimethoxyethane | 85 | II | Benzonitrile | 188 | no data |
| Isopropyl acetate | 89 | III | Dimethylsulfoxide | 189 | III |
| 1-Propanol | 97 | III | Benzyl alcohol | 205 | no data |
| 2-Butanol | 98 | III | Acetone + 5% water | | III |
| Heptane | 98 | III | EtOH + 5% water | | III |
| Water | 100 | III | IPA + 5% water | | III |
| Formic acid | 101 | III | MeCN + 5% water | | II |
| 1,4-Dioxane | 101 | II | MeOH + 5% water | | II |

Portions of crystalline DAM (each ca. 10 mg) were treated with solvents until it a solution formed, or until 1 mL had been added. The resultant samples were refrigerated (solutions) or shaken at room temperature (suspensions) for several days. The samples that had initially formed solutions were examined for signs of solid formation after refrigeration. If solid material had formed, it was analyzed by XRPD. If no solid had formed the vials were uncapped and the solutions evaporated, and solid residues analyzed by XRPD. For samples which had initially formed suspensions - if solid was still present it was analyzed by XRPD. The supernatant from the suspension was filtered and then the filtrates were then treated as the solutions above. In some experiments further solid was obtained from evaporation of the filtrates and was analyzed by XRPD where the quantity of material allowed.

The results are summarized in Table 4and Table 5 below.

**Table 4 (experiments 1-24)**

| **Solvent** | **Volume/µL** | **Initial observation** | **Result** |
|---|---|---|---|
| Diethyl ether | 1000 | Suspension | Pattern 1 |
| Pentane | 1000 | Suspension | Pattern 1 |
| Ethyl formate | 50 | Soluble | Pattern 3 |
| t-Butylmethyl ether | 1000 | Suspension | Pattern 1 |
| Acetone | 1000 | Soluble | Pattern 1, Pattern 1 |
| Methyl acetate | 1000 | Suspension | Pattern 1, Pattern 1 |
| Chloroform | 50 | Soluble | Pattern 1 |
| Methanol | 25 | Soluble | Pattern 1 |
| Tetrahydrofuran | 1000 | Suspension | Pattern 1, Pattern 1 |
| Diisopropyl ether | 1000 | Suspension | Pattern 1 |
| Ethyl acetate | 1000 | Suspension | Pattern 1, Pattern 1 |
| Ethanol | 25 | Soluble | Pattern 1 |
| Methylethyl ketone | 800 | Soluble | Pattern 1 |
| Acetonitrile | 200 | Soluble | Pattern 1, Pattern 1 |
| 2-Propanol | 50 | Soluble | Pattern 1 |
| t-Butanol | 100 | Soluble | Pattern 1 |
| 1,2-Dimethoxyethane | 1000 | Suspension | Pattern 1, Pattern 1 |
| Isopropyl acetate | 1000 | Suspension | Pattern 1 |
| 1-Propanol | 25 | Soluble | Pattern 1 |
| 2-Butanol | 50 | Soluble | Pattern 1 |
| heptane | 1000 | Suspension | Pattern 1 |
| 1,4-Dioxane | 1000 | Suspension | Pattern 1, Pattern 1 |
| Water | 20 | Soluble | Pattern 1 |
| Formic acid | 25 | Soluble | Syrup |

Most of the experiments with the first 24 solvents gave solid material giving XRPD diffractograms consistent with Pattern 1. Ethyl formate gave a new pattern (designated Pattern 3) and formic acid gave a syrup after evaporation of the experiment. Further data collected for Pattern 3 suggested it was a mixture of formate and acetate salts of denatonium.

Experiments 25-48 gave solid material with XRPD diffractograms consistent with Pattern 1 from nearly all the solvents. Three samples remained as solutions even after evaporation for six weeks. A new pattern, designated as Pattern 4, was obtained from 2-methoxyethanol.

**Table 5 (experiments 25-48)**

| **Solvent** | **Volume/µL** | **Initial observation** | **Result** |
|---|---|---|---|
| Propyl acetate | 1000 | Suspension | Pattern 1, Pattern 1 |
| 2-Pentanone | 1000 | Suspension | Pattern 1 |
| 2-Methyl-1-Propanol | 50 | Soluble | Pattern 1 |
| Toluene | 1000 | Suspension | Pattern 1 |
| 1-Butanol | 50 | Soluble | Pattern 1 |
| Isobutyl acetate | 1000 | Suspension | Pattern 1 |
| 4-Methyl-2-pentanone | 1000 | Suspension | Pattern 1, Pattern 1 |
| Acetic acid | 25 | Soluble | Pattern 1 |
| 2-Methoxyethanol | 25 | Soluble | Pattern 4 |
| Butyl acetate | 1000 | Suspension | Pattern 1 |
| Methylbutyl ketone | 1000 | Suspension | Pattern 1 |
| 3-Methyl-1-Butanol | 50 | Soluble | Pattern 1 |
| 2-Ethoxyethanol | 25 | Soluble | Pattern 1 |
| 1-Pentanol | 50 | Soluble | Pattern 1 |
| Cumene | 1000 | Suspension | Pattern 1 |
| Anisole | 750 | Suspension | Pattern 1 |
| Dimethylsulfoxide | 100 | Solution | Remained a solution |
| Benzonitrile | 500 | Nearly all soluble | Remained a solution |
| Benzyl alcohol | 25 | Solution | Remained a solution |
| Acetone + 5% water | 50 | Solution | Pattern 1, Pattern 1 |
| EtOH + 5% water | 25 | Solution | Pattern 1 |
| IPA + 5% water | 50 | Solution | Pattern 1 |
| MeCN + 5% water | 50 | Solution | Pattern 1 |
| MeOH + 5% water | 25 | Solution | Pattern 1 |

Pattern 2 was obtained from the attempted preparation of amorphous material by lyophilization of a solution of DAM in t-butanol to give DAM, lot:ED21356-005-001-01. Characterization data is summarized in Table 6 below.

**Table 6 DAM Pattern 2**

| | |
|---|---|
| CRL Batch reference | ED21356-005-001-01 |
| Appearance | White solid |
| ¹H NMR | Consistent with structure, contains t-butanol (0.11 equivalents) |
| UPLC purity | 99.5%, shows [M]⁺ 325.3 consistent with C₂₁H₂₉N₂O⁺ |
| XRPD | Pattern 2 |
| DSC | DSC of ED21356-005-001-01 at 10 °C/min shows broad endothermic event of onset 61 °C, followed by overlapping events between 78-147 °C and then further events |
| TGA | TGA of ED21356-005-001-01 at 10 °C/min shows mass losses of 2.4% over 40-85 °C, 12.4% over 87-141 °C, 15.7% over 143-180 °C followed by further mass losses |
| Storage in closed vial for 7 weeks | Conversion to Pattern 1 by XRPD |

Pattern 3 was obtained from the polymorph screen by evaporation of a solution of DAM in ethyl formate. ¹H NMR analysis shows a reduced peak for acetate and an additional peak for formate. This would be consistent with a mixture of formate and acetate salts of denatonium. Characterization data is summarized in Table 7 below.

**Table 7 DAM 7 Pattern 3**

| | |
|---|---|
| CRL Batch reference | ED21356-008-003-02 |
| Appearance | White solid |
| ¹H NMR | Consistent with a mixture of formate and acetate salts of denatonium. Reduced peak for acetate and additional peak for formate |
| UPLC purity | Purity 98% (Shows M+ ion consistent with quaternary component) Counterion not detected. |
| XRPD | Pattern 3 |
| DSC | DSC of ED21356-008-003-02 at 10 °C/min shows broad unresolved endothermic events of onset 75 °C and 93 °C, followed by further events |
| TGA | TGA of ED21356-008-003-02 at 10 °C/min shows mass losses of 2.8% over 39-121 °C and 37.6% over 121-198 °C |

Pattern 4 was obtained from the polymorph screen by evaporation of a solution of DAM in 2-methoxyethanol. A sample reanalyzed by XRPD after storage in a closed vial for 6 weeks shows conversion to Pattern 1. Characterization data is summarized below in Table 8.

**Table 8 DAM Pattern 4**

| | |
|---|---|
| CRL Batch reference | ED21356-008-033-01 |
| Appearance | White solid |
| ¹H NMR | Consistent with structure |
| UPLC purity | 99.5%, shows [M]⁺ 325.3 consistent with C₂₁H₂₉N₂O⁺ |
| XRPD | Pattern 4 |
| DSC | DSC of ED21356-008-033-01 at 10 °C/min shows a broad shallow endothermic event between 74-127 °C, followed by an endothermic event of onset 133 °C, followed by further events |
| TGA | TGA of ED21356-008-033-01 at 10 °C/min shows mass losses of 2.4% over 39-131 °C and 23.9% over 133-186 °C |
| Storage in closed vial for 6 weeks | Conversion to Pattern 1 by XRPD |

Pattern 5 was obtained by storage of DAM (Pattern 1) at 0% RH, drying *in vacuo* at room temperature or heating to 120 °C in the DSC and crash cooling. Pattern 5 converted to Pattern 1 after standing on XRPD disc under ambient lab conditions for 4 hours. A sample left overnight on XRPD disc under ambient lab conditions was also found to convert to Pattern 1. A sample stored in a closed vial for 6 weeks had also converted to Pattern 1. A summary of the characterization data for the samples of Pattern 5 can be found in Table 9.

**Table 9 DAM Pattern 5**

| | |
|---|---|
| CRL Batch references | ED21356-001-001-08, ED21356-004-001-00, ED21356-011-001-00 |
| Appearance | White solids |
| UPLC purity (ED21356-001-001-08) | 99.5%, shows [M]⁺ 325.3 consistent with C₂₁H₂₉N₂O⁺ |
| XRPD | Pattern 5 |
| ED21356-001-001-08 | |
| ED213 56-004-001-00 | |
| ED21356-011-001-00 | |
| DSC (ED21356-001-001-08) | DSC of ED21356-001-001-08 at 10 °C/min shows small broad endothermic event between 97-126 °C and endothermic event of onset 133 °C (peak 137 °C) (probable melt), followed by further events |
| TGA (ED21356-001-001-08) | TGA of ED21356-001-001-08 at 10 °C/min shows mass loss of 5.4% over 44-153 °C, followed by further mass loss |
| Storage in closed vial for 6 weeks (ED21356-004-001-01) | Conversion to Pattern 1 by XRPD |
| Standing at RT overnight on disc (ED21356-011-002-00) | Conversion to Pattern 1 by XRPD |
| Standing at RT on disc for 4 hours (ED21356-001-001-09) | Conversion to Pattern 1 by XRPD |

DAM was highly crystalline with an XRPD Pattern designated as Pattern 1. During the investigations into DAM there were 4 additional XRPD Patterns found. One of these (Pattern 3) was found to be a mixture of denatonium acetate and formate salts based upon the ¹H NMR and is therefore not a pattern representative of denatonium acetate alone. Pattern 2 was obtained from the attempted preparation of amorphous material by lyophilization of a solution of DAM in t-butanol. After storage for 7 weeks in a closed vial it was observed to have converted to Pattern 1. Pattern 4 was obtained from the polymorph screen by evaporation of a solution of DAM in 2-methoxyethanol. A sample reanalyzed by XRPD after storage in a closed vial for 6 weeks showed conversion to Pattern 1. Pattern 5 was obtained by storage of DAM (Pattern 1) at 0% RH, drying *in vacuo* at room temperature or heating to 120 °C in the DSC and crash cooling. It was observed to readily convert to Pattern 1 on standing on the XRPD disc under ambient lab conditions after 4 hours.

## Claims

1. A denatonium acetate monohydrate salt of structural formula 1: 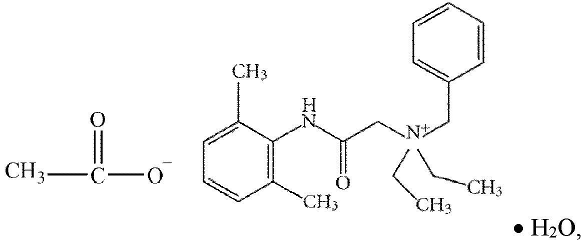 wherein the denatonium acetate monohydrate salt is a crystalline monohydrate.

2. The salt of claim 1, which is **characterized by** an X-ray powder diffraction (XRPD) spectrum substantially as shown in Fig. 5A or Fig. 5B.

3. A pharmaceutical composition comprising a therapeutically effective amount of the salt according to any one of claims 1-2 in association with one or more pharmaceutically acceptable carriers.

4. The pharmaceutical composition of claim 3, wherein the one or more pharmaceutically acceptable carriers comprise a biocompatible polymer.

5. The pharmaceutical composition of claim 4, wherein the biocompatible polymer is cellulose.

6. The pharmaceutical composition of any one of claims 3-5, wherein the one or more pharmaceutically acceptable carriers comprise a saccharide.

7. The pharmaceutical composition of claim 6, wherein the saccharide is a sugar alcohol.

8. The pharmaceutical composition of claim 7, wherein the sugar alcohol is mannitol.

9. The pharmaceutical composition of any one of claims 3-8, wherein the one or more pharmaceutically acceptable carriers comprise talc.

10. The pharmaceutical composition of any one of claims 3-9, wherein the one or more pharmaceutically acceptable carriers comprise an organic acid.

11. The pharmaceutical composition of claim 10, wherein the organic acid is acetic acid.

12. The pharmaceutical composition of any one of claims 3-11, wherein the pharmaceutical composition is formulated for oral administration.

13. The pharmaceutical composition of any one of claims 3-11, wherein the pharmaceutical composition comprises solid granules.

14. A process for preparing a denatonium acetate monohydrate salt of structural formula 1: 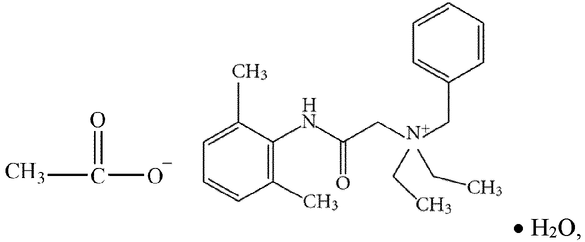 the process comprising contacting anhydrous denatonium acetate with an alkyl isobutyl ketone and water, resulting in formation of a solid phase comprising denatonium acetate monohydrate, wherein the alkyl isobutyl ketone is a C₁₋₃ alkyl isobutyl ketone.

15. The process of claim 14, comprising the steps of (1) contacting an equivalent of DAA (denatonium acetate, anhydrous) with methyl isobutyl ketone and water such that the water concentration is above 10 weight percent, (2) recovering a resultant solid phase, and (3) removing the solid phase therefrom.

16. The process of claim 14, wherein the alkyl isobutyl ketone is methyl isobutyl ketone or ethyl isobutyl ketone.

17. The process of any one of claims 14-16, wherein the denatonium acetate monohydrate is a crystalline monohydrate.

18. The process of claim 17, wherein the crystalline monohydrate is **characterized by** an X-ray powder diffraction (XRPD) spectrum substantially as shown in Fig. 5A or Fig. 5B.

## Patentansprüche

1. Denatoniumacetat-Monohydrat-Salz der Strukturformel 1: 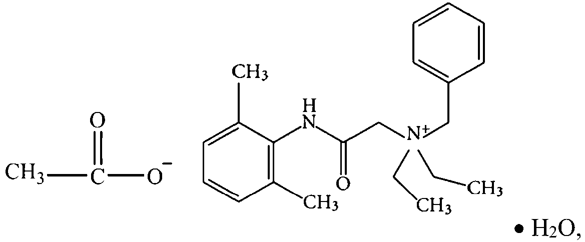 wobei das Denatoniumacetat-Monohydrat-Salz ein kristallines Monohydrat ist.

2. Salz nach Anspruch 1, das durch ein durch Röntgenpulverdiffraktometrie (XRPD) erzeugtes Spektrum gekennzeichnet ist, das im Wesentlichen dem in Abb. 5A oder Abb. 5B gezeigten Spektrum entspricht.

3. Pharmazeutische Zusammensetzung umfassend eine therapeutisch wirksame Menge des Salzes nach einem der Ansprüche 1 bis 2 in Verbindung mit einem oder mehreren pharmazeutisch verträglichen Trägerstoffen.

4. Pharmazeutische Zusammensetzung nach Anspruch 3, wobei der eine oder die mehreren pharmazeutisch verträglichen Trägerstoffe ein biokompatibles Polymer umfassen.

5. Pharmazeutische Zusammensetzung nach Anspruch 4, wobei das biokompatible Polymer Cellulose ist.

6. Pharmazeutische Zusammensetzung nach einem der Ansprüche 3 bis 5, wobei der eine oder die mehreren pharmazeutisch verträglichen Trägerstoffe ein Saccharid umfassen.

7. Pharmazeutische Zusammensetzung nach Anspruch 6, wobei das Saccharid ein Zuckeralkohol ist.

8. Pharmazeutische Zusammensetzung nach Anspruch 7, wobei der Zuckeralkohol Mannitol ist.

9. Pharmazeutische Zusammensetzung nach einem der Ansprüche 3 bis 8, wobei der eine oder die mehreren pharmazeutisch verträglichen Trägerstoffe Talkum umfassen.

10. Pharmazeutische Zusammensetzung nach einem der Ansprüche 3 bis 9, wobei der eine oder die mehreren pharmazeutisch verträglichen Trägerstoffe eine organische Säure umfassen.

11. Pharmazeutische Zusammensetzung nach Anspruch 10, wobei die organische Säure Essigsäure ist.

12. Pharmazeutische Zusammensetzung nach einem der Ansprüche 3 bis 11, wobei die pharmazeutische Zusammensetzung zur oralen Verabreichung formuliert ist.

13. Pharmazeutische Zusammensetzung nach einem der Ansprüche 3 bis 11, wobei die pharmazeutische Zusammensetzung feste Granulate umfasst.

14. Verfahren zur Herstellung eines Denatoniumacetat-Monohydrat-Salzes der Strukturformel 1: 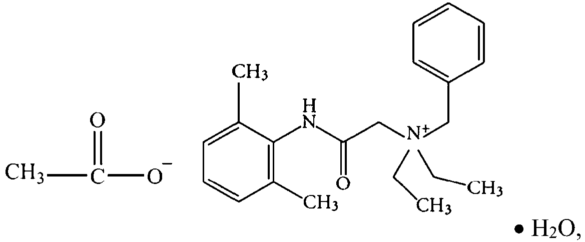 wobei das Verfahren das Inkontaktbringen von wasserfreiem Denatoniumacetat mit Alkylisobutylketon und Wasser umfasst, was zur Bildung einer festen Phase führt, die Denatoniumacetat-Monohydrat umfasst, wobei das Alkylisobutylketon ein C₁₋₃-Alkylisobutylketon ist.

15. Verfahren nach Anspruch 14, umfassend die Schritte: (1) Inkontaktbringen eines Äquivalents DAA (Denatoniumacetat, wasserfrei) mit Methylisobutylketon und Wasser, so dass die Wasserkonzentration über 10 Gewichtsprozent liegt, (2) Gewinnen einer resultierenden festen Phase und (3) Entfernen der festen Phase davon.

16. Verfahren nach Anspruch 14, wobei das Alkylisobutylketon Methylisobutylketon oder Ethylisobutylketon ist.

17. Verfahren nach einem der Ansprüche 14 bis 16, wobei das Denatoniumacetat-Monohydrat ein kristallines Monohydrat ist.

18. Verfahren nach Anspruch 17, wobei das kristalline Monohydrat durch ein durch Röntgenpulverdiffraktometrie (XRPD) erzeugtes Spektrum gekennzeichnet ist, das im Wesentlichen dem in Abb. 5A oder Abb. 5B gezeigten Spektrum entspricht.

## Revendications

1. Sel de monohydrate d'acétate de dénatonium de formule structurale 1 : 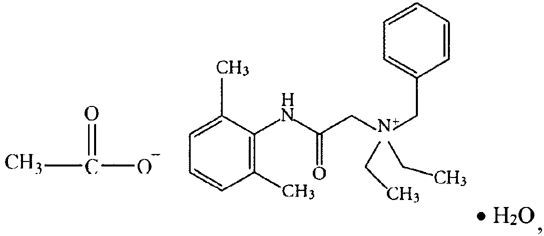 le sel de monohydrate d'acétate de dénatonium étant un monohydrate cristallin.

2. Sel selon la revendication 1, qui est **caractérisé par** un spectre de diffraction des rayons X sur poudre (XRPD) essentiellement tel que représenté sur la figure 5A ou la figure 5B.

3. Composition pharmaceutique comprenant une quantité thérapeutiquement efficace du sel selon l'une quelconque des revendications 1 à 2 en association avec un ou plusieurs véhicules pharmaceutiquement acceptables.

4. Composition pharmaceutique selon la revendication 3, les un ou plusieurs véhicules pharmaceutiquement acceptables comprenant un polymère biocompatible.

5. Composition pharmaceutique selon la revendication 4, le polymère biocompatible étant la cellulose.

6. Composition pharmaceutique selon l'une quelconque des revendications 3 à 5, les un ou plusieurs véhicules pharmaceutiquement acceptables comprenant un saccharide.

7. Composition pharmaceutique selon la revendication 6, le saccharide étant un alcool de sucre.

8. Composition pharmaceutique selon la revendication 7, l'alcool de sucre étant le mannitol.

9. Composition pharmaceutique selon l'une quelconque des revendications 3 à 8, les un ou plusieurs véhicules pharmaceutiquement acceptables comprenant du talc.

10. Composition pharmaceutique selon l'une quelconque des revendications 3 à 9, les un ou plusieurs véhicules pharmaceutiquement acceptables comprenant un acide organique.

11. Composition pharmaceutique selon la revendication 10, l'acide organique étant l'acide acétique.

12. Composition pharmaceutique selon l'une quelconque des revendications 3 à 11, la composition pharmaceutique étant formulée pour une administration orale.

13. Composition pharmaceutique selon l'une quelconque des revendications 3 à 11, la composition pharmaceutique comprenant des granulés solides.

14. Procédé de préparation d'un sel de monohydrate d'acétate de dénatonium de formule structurale 1 : 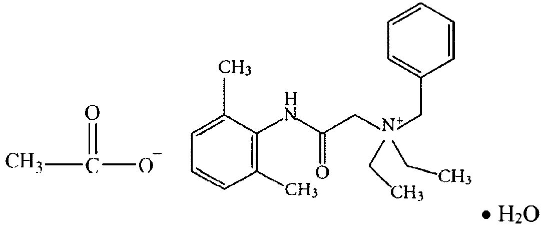 le procédé comprenant la mise en contact d'acétate de dénatonium anhydre avec une alkylisobutylcétone et de l'eau, résultant en la formation d'une phase solide comprenant du monohydrate d'acétate de dénatonium, l'alkylisobutylcétone étant une alkyle en C₁₋₃-isobutylcétone.

15. Procédé selon la revendication 14, comprenant les étapes consistant à (1) mettre en contact un équivalent de DAA (acétate de dénatonium, anhydre) avec de la méthylisobutylcétone et de l'eau de telle sorte que la concentration en eau soit supérieure à 10 pour cent en poids, (2) récupérer une phase solide résultante, et (3) en retirer la phase solide.

16. Procédé selon la revendication 14, l'alkylisobutylcétone étant la méthylisobutylcétone ou l'éthylisobutylcétone.

17. Procédé selon l'une quelconque des revendications 14 à 16, le monohydrate d'acétate de dénatonium étant un monohydrate cristallin.

18. Procédé selon la revendication 17, le monohydrate cristallin étant **caractérisé par** un spectre de diffraction des rayons X sur poudre (XRPD) essentiellement tel que représenté sur la figure 5A ou la figure 5B.
